(19) Europäisches Patentamt

European Patent Office

Office européen des brevets

(11) **EP 2 292 822 B1**

(12) **EUROPEAN PATENT SPECIFICATION**

(45) Date of publication and mention of the grant of the patent:
**03.07.2013 Bulletin 2013/27**

(21) Application number: **09754613.9**

(22) Date of filing: **21.05.2009**

(51) Int Cl.:
*D04H 3/16* (2006.01)  *A61F 13/15* (2006.01)
*A61F 13/511* (2006.01)  *A61F 13/53* (2006.01)
*D04H 3/00* (2012.01)  *D01D 5/08* (2006.01)
*D01D 5/098* (2006.01)  *D04H 3/14* (2012.01)
*D01F 6/70* (2006.01)  *D01F 6/04* (2006.01)
*A61F 13/537* (2006.01)

(86) International application number:
**PCT/JP2009/059346**

(87) International publication number:
**WO 2009/145105 (03.12.2009 Gazette 2009/49)**

(54) **FILAMENT-MIXED SPUN-BONDED NONWOVEN FABRIC AND USE THEREOF**

SPINNVLIESSSTOFF UND SEINE VERWENDUNG

TISSU NON TISSÉ FILÉ-LIÉ À FILAMENTS MÉLANGÉS ET SON UTILISATION

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **29.05.2008  JP 2008141461**
**26.01.2009  JP 2009014661**

(43) Date of publication of application:
**09.03.2011  Bulletin 2011/10**

(73) Proprietor: **Mitsui Chemicals, Inc.**
**Tokyo 105-7117 (JP)**

(72) Inventor: **KUNIMOTO, Naosuke**
**Sodegaura-shi**
**Chiba 299-0265 (JP)**

(74) Representative: **HOFFMANN EITLE**
**Patent- und Rechtsanwälte**
**Arabellastrasse 4**
**81925 München (DE)**

(56) References cited:
WO-A1-2007/138733    DE-A1- 2 050 371
JP-A- 9 119 050        JP-A- 2004 244 791
JP-A- 2006 188 804     JP-A- 2006 188 804
JP-A- 2007 009 403

**Description**

TECHNICAL FIELD

The present invention relates to a mixed fiber spun bonded nonwoven fabric which has excellent bulkiness, initial hydrophilicity, long-lasting hydrophilicity, flexibility, resistance to fluff, stretchability and touch and low stickiness, and is suitable for a surface sheet and/or a second sheet or a sheet for lapping an absorber (core lap) of absorbent articles such as sanitary napkins, panty liners, incontinence pads, disposable diapers and other absorbent articles.

TECHNICAL BACKGROUND

Since nonwoven fabrics have excellent gas permeability and flexibility, they have been widely used for surface sheets of absorbent articles such as disposable diapers, sanitary napkins and the like which need to have surface properties such as absorbing properties capable of quickly transferring liquids excreted or discharged such as blood, urine or the like to absorbers, a soft surface touched to the skin of the wearer and low irritation to the skin.

As a method of improving the skin touch properties and wet backing properties concerning to surface sheets, Patent document 1 discloses a method such that a high shrinkable fiber sheet and a low shrinkable or non-shrinkable nonwoven fabric are laminated to prepare a unit and the high shrinkable fiber sheet is shrunk by heat treatment to form wrinkles on the surface. Patent document 2 discloses a method of laminating a bulky nonwoven fabric obtainable by endowing bulkiness with hot air treatment and a shrink potential-having fiber nonwoven fabric capable of being shrunk by heat treatment.

Furthermore, as a method for improving absorption of liquid such as urine and the like discharged and a transferring rate thereof, Patent document 3 discloses a method of preparing a nonwoven fabric having a complex phase structure with a low water permeating part that has a bulky layered structure of a hydrophobic fiber layer and a hydrophilic fiber layer, and a water permeating part that a hydrophobic fiber and a hydrophilic fiber are mingled. Patent document 4 discloses a method of preparing a nonwoven fabric by mixing a hydrophilic fiber and a water repellent fiber.

The method of endowing bulkiness by heat treatment for a nonwoven fabric, however, has a complicated process and a risk of lowering in flexibility and touch because the heat treatment occasionally increases the fiber diameter of the fibers forming the nonwoven fabric by heat shrinkage without thinning the fiber diameter. Moreover, since the nonwoven fabric obtainable by mixing a hydrophilic fiber and a water repellent fiber is insufficient in bulkiness, a surface sheet having both of flexibility and bulkiness has not been obtained.

Patent document 5 describes a spunbond nonwoven fabric comprising mixed fibers of a polyolefin and an elastomer produced by extruding them simultaneously on the same die. This nonwoven fabric has excellent touch and stretchability.

In Patent document 6 non-woven fabric laminate and a method for producing the non-woven fabric laminate has been described. The nonwoven laminate includes at least one meltblown nonwoven fabric layer and mixed-fiber spunbonded nonwoven fabric layers on both surfaces of at least one meltblown nonwoven fabric layer.

Patent document 7 discloses spunbonded fabrics with hydrophilic properties from thermoplastic filaments. Patent document 8 shows that a hydrophobic polymer and a hydrophilic polymer are meltblown from a meltblowing spinneret where the orifices for the hydrophobic polymer and those for the hydrophilic polymer are alternately arranged in line to give a combined filament yarn nonwoven fabric, which is treated with a hydrophilizing agent to give the objective combined filament yarn nonwoven fabric.

Patent document 9 discloses many methods of adding a nonionic surface-active agent such as polyoxyethylene alkylether and the like as a means of hydrophilizing a nonwoven fabric such as a spun-bonded nonwoven fabric composed of a propylene polymer. It is found that the addition of the surface-active agent improves the initial hydrophilicity but the long-lasting hydrophilicity is inferior. The property "initial hydrophilicity" indicates hydrophilicity in a general broad sense. In the present specification, the initial hydrophilicity is optionally used in order to distinguish from long-lasting hydrophilicity. The long-lasting hydrophilicity indicates hydrophilicity after exposing to environment at a temperature rather higher than room temperature (about 40°C) for a certain period of time. In the present specification, the term "long-lasting hydrophilicity" is referred hereinafter.

PRIOR ARTS

PATENT DOCUMENTS

Patent document 1: JP-A-H6 (1994)-128853

Patent document 2: JP-A-2003-250836
Patent document 3: JP-A-2002-20957
Patent document 4: JP-A-2004-73759
Patent document 5: JP-A-2007-009403
Patent document 6: WO 2007/138733 A1
Patent document 7: DE-20 50 371 A1
Patent document 8: JP 9 119050 A
Patent document 9: JP-A-2006-188804

SUMMARY OF THE INVENTION

SUBJECT TO BE SOLVED BY THE INVENTION

It is an object of the present invention to provide a mixed fiber spun-bonded nonwoven fabric which has excellent bulkiness, long-lasting hydrophilicity, initial hydrophilicity, flexibility, resistance to fluff, stretchability and touch and low stickiness and is suitable for a surface sheet and/or a second sheet or a sheet for lapping an absorber (core lap) of absorbent articles such as sanitary napkins, panty liners, incontinence pads, disposable diapers and other absorbent articles.

In order to solve the subject, the present inventors have been studied variously and found that a hydrophilized thermoplastic resin long fiber is mixed with a thermoplastic elastomer long fiber and thereby the subject can be solved.

MEANS FOR SOLVING THE SUBJECT

The present invention provides a mixed fiber spun-bonded nonwoven fabric which comprises 90 to 10 % by weight of a long fiber type thermoplastic resin (A) and 10 to 90 % by weight of a long fiber type thermoplastic elastomer (B) wherein at least, the long fiber type thermoplastic resin (A) is hydrophilized. The present invention further provides a surface sheet and a second sheet for absorbent articles which sheets comprise the mixed fiber spun-bonded nonwoven fabric, and provides absorbent articles.

EFFECT'OF THE INVENTION

The mixed fiber spun bonded nonwoven fabric of the present invention has excellent bulkiness, initial hydrophilicity, long-lasting hydrophilicity, flexibility, resistance to fluff, stretchability and touch and low stickiness so that it is suitable for a surface sheet and/or a second sheet for absorbent articles.

BRIEF DESCRIPTION OF DRAWING

Fig. 1 is a schematic view of a specimen for measuring a fiber diameter of a (mixed fiber) spun bonded nonwoven fabric.

Fig. 2 is 40-power and 200-power micrographs of a part where the specimen is folded as shown in Fig. 1.

EMBODIMENT FOR CARRYING OUT THE INVENTION

<Thermoplastic resin (A)>

As a thermoplastic resin (A) that is a raw material of a long fiber type thermoplastic resin, which is one of components forming the mixed fiber spun bonded nonwoven fabric of the present invention, known various thermoplastic resins can be used. The thermoplastic resin (A) is a resinous polymer different from the thermoplastic elastomer (B) as described later and is usually a crystalline polymer having a melting point (Tm) of not lower than 100°C or a non-crystalline polymer having a glass transition temperature of not lower than 100°C. Among these thermoplastic resins (A), the crystalline thermoplastic resin is preferred.

Moreover, among the thermoplastic resins (A), it is preferred to use a thermoplastic resin (extensible thermoplastic resin) having very low elastic recovery and such a property that a nonwoven fabric obtainable by a known method of producing a spun bond nonwoven fabric using said thermoplastic resin has an elongation at the maximum point of not less than 50%, preferably not less than 70%, more preferably not less than 100%. In using a mixed fiber spun bond nonwoven fabric obtainable by mixing the extensible thermoplastic resin and the long fiber type thermoplastic elastomer (B) as a surface sheet, the long fiber type extensible thermoplastic resin and the long fiber type thermoplastic elastomer (B) are stretched by stretch processing the mixed fiber spun bond nonwoven fabric, thereafter, when the stress is released, only the elasticity of the long fiber type thermoplastic elastomer (B) is recovered and the long fiber type extensible thermoplastic resin elongated is folded without elastic recovery and thereby the bulkiness is arisen in the mixed fiber spun bond nonwoven fabric and also the long fiber type extensible thermoplastic resin is thinned by the elongation to have improved flexibility and touch and also to have a function of suppressing elongation. The upper limit of the elongation at the maximum point of the spun bond nonwoven fabric made of the thermoplastic resin (A), which is not particularly limited, is usually not more than 300%.

Examples of the thermoplastic resin (A) are a polyolefin such as a homopolymer or a copolymer of $\alpha$-olefins including ethylene, propylene, 1-butene, 1-hexene, 4-methyl-1-pentene, 1-octene and the like, the polyolefin being for example high pressure low density polyethylene, linear low density polyethylene (namely LLDPE), high density polyethylene (namely HDPE), polypropylene (propylene homopolymer), polypropylene random copolymer, poly-1-butene, poly-4-methyl-1-pentene, ethylene/propylene random copolymer, ethylene/1-butene random copolymer, propylene/1-butene random copolymer, etc; a polyester such as polyethylene terephthalate, polybutylene terephthalate, polyethylene naphthalate and the like; a polyamide such as nylon-6, nylon-66, polymethaxylene adipamide, and the like; polyvinyl chloride, polyimide, ethylene/vinyl acetate copolymer, ethylene/vinyl alcohol copolymer, ethylene/(meth)acrylic acid copolymer, ethylene/acrylic acid ester/carbon monoxide copolymer, polyacrylonitrile, polycarbonate, polystyrene, ionomer and mixtures thereof. Of these, high-pressure low density polyethylene, linear low density polyethylene, high density polyethylene, a propylene polymer such as polypropylene or polypropylene random copolymer, polyethylene terephthalate and polyamide are more preferred.

Of these thermoplastic resins (A), propylene polymers are particularly preferred in the viewpoint of spinning stability at molding or stretching processability of a nonwoven fabric.

Preferable examples of the propylene polymer are a propylene homopolymer having a melting point (Tm) of not lower than 135°C, and a copolymer of propylene and not more than 10 mol% of one or two or more $\alpha$-olefins having 2 or more carbon atoms (excluding 3 carbon atoms), preferably 2 to 8 carbon atoms (excluding 3 carbon atoms), such as ethylene, 1-butene, 1-pentene, 1-hexene, 1-octene, 4-methyl-1-pentene, etc.

Of these propylene polymers, a propylene/$\alpha$-olefin random copolymer having a melting point of 135 to 155°C is preferred because mixed fiber spun bonded nonwoven fabrics having excellent stretchability, initial hydrophilicity, long-lasting hydrophilicity, flexibility and touch can be prepared.

The melt flow rate (MFR: ASTMD-1238, 230°C under a load of 2160 g) of the propylene polymer is not particularly limited as long as melt spinning thereof can be carried out. The melt flow rate is usually 1 to 1000 g/10 min, preferably 5 to 500 g/10 min, more preferably 10 to 100 g/10 min. Furthermore, in the propylene polymer of the present invention, the ratio of weight average molecular weight (Mw) to number average molecular weight (Mn) (Mw/Mn) is usually 1.5 to 5.0. The ratio is further preferably 1.5 to 3.0 because the spinning properties are good and fibers having particularly excellent fiber strength can be prepared. Mw and Mn can be determined using GPC (Gel permeation chromatography) by a known method.

In the view point of spinning properties and stretching processability, an olefin polymer composition obtainable by adding a small amount, preferably 1 to 20 % by weight, more preferably 2 to 15 % by weight, furthermore preferably 4 to 10 % by weight of a high density polyethylene (HDPE) to the propylene polymer is preferred provided that the total amount of the propylene polymer and HDPE is 100 % by weight, because the resulting nonwoven fabric laminate can have more improved stretching processability.

The HDPE for adding to the propylene polymer has a density, which is particularly limited, of usually 0.94 to 0.97 g/cm$^3$, preferably 0.95 to 0. 97 g/cm$^3$, more preferably 0.96 to 0.97 g/cm$^3$. The HDPE has a melt flow rate (MFR: ASTMD-1238, 190°C under a load of 2160 g), which is not particularly limited as long as it has spinnability, of usually 0.1 to 100 g/10 min, more preferably 0.5 to 50 g/10 min, furthermore preferably 1 to 30 g/ 10 min in the viewpoint of exhibition of extensibility. In the present invention, the term "good spinnability" indicates the fact that fiber breakage is not caused at the time of outputting fiber from a spinning nozzle and during stretching, and filament fusing is not caused.

<Thermoplastic elastomer (B)>

As the thermoplastic elastomer (B), which is one component forming the mixed fiber spun bonded nonwoven fabric of the present invention, various known thermoplastic elastomers can be used, and two or more thermoplastic elastomers may be used simultaneously. Examples thereof are:

styrene elastomers comprising block copolymers obtainable by a polymer block made of at least one aromatic vinyl compound such as styrene and the like and a polymer block made of at least one conjugated diene compound such as butadiene, isoprene and the like, or hydrogenated products of the block copolymer, such as polystyrene-polybutadiene-polystyrene block copolymer (referred to SBS), polystyrene-polyisoprene- polystyrene block copolymer (referred to SIS), and their hydrogenated products i.e. polystyrene-poly/ethylene/butylene-polystyrene block copolymer (referred to SEBS) and polystyrene-poly/ethylene/propylene-polystyrene block copolymer (referred to SEPS); polyester elastomers typified by a block copolymer made of a high crystalline aromatic polyester and a non-crystalline aliphatic polyether;

polyamide elastomers typified by a block copolymer made of a crystalline polyamide having a high melting point and a non-crystalline polyether or polyester having a low glass transition temperature (Tg);

thermoplastic polyurethane elastomers typified by a block copolymer made of as a hard segment a polyurethane and as a soft segment a polycarbonate polyol, an ether polyol, a caprolactone polyester or an adipate polyester;

polyolefin elastomers typified by a non-crystalline or low-crystalline ethylene/$\alpha$-olefin random copolymer, propylene/$\alpha$-olefin random copolymer or propylene/ethylene/$\alpha$-olefin random copolymer, or mixture of the non-crystalline or low-crystalline random copolymer and a propylene homopolymer or a crystalline polyolefin such as a copolymer of propylene and a small amount of an $\alpha$-olefin, high density polyethylene, middle density polyethylene;

vinyl chloride elastomers; and

fluorine elastomers.

Examples of the styrene elastomers are diblock and triblock copolymers obtainable by using, as a base, polystyrene block and butadiene rubber block or isoprene rubber block. The rubber block may be unsaturated or hydrogenated completely. Examples of the styrene elastomer are KRATON POLYMER (Trade Name: available by Schell Chemicals Ltd.), SEPTON (Trade Name: available by Kuraray Co. Ltd.), TUFTEC (Trade Name: available by Asahi Kasei Co.) and LEOSTOMER (Trade Name: available by Riken technos. Co.).

Examples of the polyester elastomer are HYTREL (Trade Name: available by E.I. Dupont Co.) and PELPRENE (Trade Name: available by TOYOBO. CO., Ltd.).

An example of the amide elastomer is PEBAX (Trade Name: available by Atofina Japan).

Examples of the polyolefin elastomer are an ethylene/$\alpha$-olefin copolymer and a propylene/$\alpha$-olefin copolymer. Specific examples thereof are TAFMER (Trade Name: available by Mitsui Chemicals Inc.), VISTMAXX (Trade Name: available by Exxon Mobil Inc. Engage, which is an ethylene-octene copolymer (Trade Name: available by DuPont Dow Elastomers Inc.) and CATALLOY containing a crystalline olefin copolymer (Trade Name: available by Montel Inc.).

Examples of the vinyl chloride elastomer are Leonyl (Trade Name: available by Riken Technos Co.) and Posmile (Trade Name: available by Shin-Etsu Polymer Co., Ltd.).

Of these thermoplastic elastomers (B), the polyolefin elastomer and thermoplastic polyurethane elastomer are preferred. Particularly, the thermoplastic polyurethane elastomer is preferred because of having stretchability, processability and capable of preparing mixed fiber spun bonded nonwoven fabrics having excellent initial hydrophilicity and long-lasting hydrophilicity.

<Thermoplastic polyurethane elastomer>

Of the thermoplastic polyurethane elastomers, thermoplastic polyurethane elastomers having a starting temperature for solidification of not lower than 65°C, preferably not lower than 75°C, most preferably not lower than 85°C are preferred. The upper limit of the starting temperature for solidification is preferably 195°C. The starting temperature for solidification is measured by a differential scanning calorimeter (DSC) and determined in such a way that the temperature of the thermoplastic polyurethane elastomer is increased at a rate of 10°C/min to 230°C and kept at 230°C for 5 min, and then when the temperature thereof is decreased at a rate of 10°C/min, the temperature at which an exothermic peak derived from the solidification of the resulting thermoplastic polyurethane elastomer is started is taken as the starting temperature for solidification. When the starting temperature for solidification is not lower than 65°C, it is possible to prevent defective molding such as fusion of fibers, broken fiber, massed resin in preparing mixed fiber spun bonded nonwoven fabrics, and also it is possible to prevent mixed fiber spun bonded nonwoven fabrics from winding to an emboss roller in heat emboss processing. Furthermore, the resulting mixed fiber spun bonded nonwoven fabrics have low stickiness and are suitably used for materials contacting with the skin such as clothes, hygienic materials, materials of sporting goods and the like. While, when the starting temperature for solidification is not higher than 195°C, it is possible to improve the molding processability. The starting temperature for solidification of the molded fiber tends to be higher than that of the thermoplastic polyurethane elastomer used for the fiber.

In order to regulate the starting temperature for solidification of the thermoplastic polyurethane elastomer to be not lower than 65°C, it is necessary to select a polyol, an isocyanate compound and a chain extender each having an optimum chemical structure, which are used as materials for the thermoplastic polyurethane elastomer, and to regulate the amount of a hard segment. The amount of the hard segment is a value (% by weight) determined by dividing the total weight of the isocyanate compound and the chain extender which are used in the preparation of the thermoplastic polyurethane elastomer by the total weight of the polyol, the isocyanate compound and the chain extender and multiplying the resulting value by 100. The hard segment amount is preferably 20 to 60 % by weight, more preferably 22 to 50 % by weight, most preferably 25 to 48 % by weight.

The thermoplastic polyurethane elastomer has a particle number of components insoluble in the polar solvent of preferably not more than 3, 000, 000 particles/g, more preferably not more than 2,500,000 particles/g, furthermore preferably not more than 2,000,000 particles/g. In the thermoplastic polyurethane elastomer, the components insoluble in the polar solvent are agglomerates such as fish eye, gel and the like generated during the production of the thermoplastic polyurethane elastomer. The agglomerates are components caused by the raw materials constituting the thermoplastic polyurethane elastomer and by chemical reaction between these raw materials, for example, components derived from hard segment agglomerates of the thermoplastic polyurethane elastomer and components obtainable by crosslinking the hard segment and/or the soft segment with allophanate bond or biuret bond.

The particle number of the components insoluble in the polar solvent is determined by dissolving the thermoplastic polyurethane elastomer in a dimethyl acetoamide solvent (hereinafter referred to "DMAC") and measuring the insoluble components by means of a particle size distribution measuring apparatus equipped with a 100 μm aperture utilizing an electrical sensing zone method. Using the apparatus equipped with a 100 μm aperture, the number of particles of 2 to 60 mm can be measured in terms of uncrosslinked polystyrene.

Regulating the particle number of the components insoluble in the polar solvent of not more than 3,000,000 per 1 g of the thermoplastic polyurethane elastomer, it is possible to more depress the problems in the starting temperature range for solidification of the thermoplastic polyurethane elastomer, such as increase of a distribution of a fiber diameter, fiber breakage in spinning and the like. Moreover, in the molding of nonwoven fabrics by a large-size spun bonding molding machine, from the viewpoint of depressing mingling of bubbles into a strand or occurrence of fiber breakage, the thermoplastic polyurethane elastomer has a water content of preferably not more than 350 ppm, more preferably not more than 300 ppm, most preferably not more than 150 ppm.

In the thermoplastic polyurethane elastomer, from the viewpoint of stretchability, the total sum (a) of heat of fusion determined from the endothermic peaks at a peak temperature of 90 to 140° C and the total sum (b) of heat of fusion determined from the endothermic peaks at a peak temperature of over 140°C and not higher than 220° C, as measured by a differential scanning calorimeter, preferably satisfy the following formula (I);

$$a \ / \ (a \ + \ b) \ \leq \ 0.8 \qquad (I),$$

more preferably the following formula (II);

$$a \ / \ (a \ + \ b) \ \leq \ 0.7 \qquad (II),$$

most preferably the following formula (III);

$$a \ / \ (a \ + \ b) \ \leq \ 0.55 \qquad (III).$$

The ratio of a / (a + b) means a ratio (unit : %) of heat of fusion of the hard domain of the thermoplastic polyurethane elastomer. When the ratio of heat of fusion of the hard domain of the thermoplastic polyurethane elastomer is not more than 80 %, the strength and the stretchability of fibers and nonwoven fabrics are improved in fibers, particularly mixed fiber spun bonded nonwoven fabrics. In the present invention, the lower limit of the ratio of heat of fusion of the hard domain of the thermoplastic polyurethane elastomer is preferably about 0.1 %.

The thermoplastic polyurethane elastomer has a melt viscosity, as measured at 200°at a shear rate of 100 sec[-1] of preferably 100 to 3000 Pa·s, more preferably 200 to 2000 Pa·s, most preferably 1000 to 1500 Pa·s. The melt viscosity is determined by a capirograph (manufactured by Toyo Seiki Co., Ltd., a nozzle length of 30 mm, a diameter of 1 mm).

The thermoplastic polyurethane elastomer having such properties can be obtained by, for example, the production process as described in JP-A-200-4-244791.

The thermoplastic polyurethane elastomer having a low content of the components insoluble in the polar solvent is obtainable by polymerization-reacting the polyol, the isocyanate compound and the chain extender, followed by filtration, as described later.

<Hydrophilization treating agent>

In order to endow the initial hydrophilicity and the long-lasting hydrophilicity to the mixed fiber spun bonded nonwoven fabric according to the present invention, it is necessary to at least endow hydrophilicity to the long fiber type thermoplastic resin (A). Examples of a hydrophilization-treating agent for endowing the hydrophilicity are surface-active agents and the like, and a non-ionic surface-active agent is preferred among them. Examples of the non-ionic surface-active agent are ether type non-ionic surface-active agents such as polyoxyethylene alkylether, polyoxypropylene alkylether, polyoxyethylene alkylphenylether or polyoxypropylene alkylphenylether; polyvalent alcohol ether type non-ionic surface-active agents such as alkyl glycoxide and the like; ester type non-ionic surface-active agents such as polyoxyethylene aliphatic acid ester or polyoxypropylene aliphatic acid ester; polyvalent alcohol ester type non-ionic surface-active agents such as sucrose aliphatic acid ester, sorbitane aliphatic acid ester, polyoxyethylene aliphatic acid ester or polyoxypropylene aliphatic acid ester; and amide type non-ionic surface-active agents such as aliphatic acid alkanolamide or alkylene oxide adducts of an aliphatic amide having an acyl group of 8 to 18 carbon atoms.

These non-ionic surface-active agents may be used singly or as a mixture of two or more of the non-ionic surface-active agents.

As the ether type non-ionic surface-active agents, surface-active agents having an alkyl group of 8 to 50 carbon atoms or an alkylphenyl group with an alkyl group of 8 to 18 carbon atoms are preferred.

Of these non-ionic surface-active agents, ether type non-ionic surface-active agents made from an alkylene oxide adduct of an aliphatic alcohol having 10 to 40 carbon atoms, preferably 12 to 24 carbon atoms, more preferably 16 to 22 carbon atoms (AE type non-ionic surface-active agents) and ester type non-ionic surface-active agents having an ester of an aliphatic acid of 8 to 18 carbon atoms are preferred.

As the method for hydrophilizing the long fibers of the thermoplastic resin (A), there is a method of adding the surface-active agent. Specific examples thereof are a method of applying the surface-active agent on the long fibers and a method of previously adding the surface-active agent to the thermoplastic resin (A) and fiberizing (kneading). Particularly, it is preferred to employ a method of applying the non-ionic surface-active agent on the surfaces of the long fibers of the thermoplastic resin (A) or kneading it in the thermoplastic resin (A), in an amount of 0.1 to 10 parts by weight, more preferably 0.5 to 5 parts by weight of based on 100 parts by weight of the thermoplastic resin (A). Moreover, from the viewpoint of long-lasting hydrophilicity, a method of adding the surface-active agent to the thermoplastic resin (A) and then fiberizing (kneading) is more preferred.

When the amount of the non-ionic surface agent added is less than 0.1 part by weight, the resulting mixed fiber spun bonded nonwoven fabrics likely have insufficient effects of improving the initial hydrophilicity and long-lasting hydrophilicity. On the other hand, when the amount is over 10 parts by weight, the processability is likely lowered and since the amount of the non-ionic surface-active agent soaked on the fiber surface is increased, the resulting mixed fiber spun bonded nonwoven fabrics are likely sticky.

Since the mixed fiber spun bonded nonwoven fabrics having excellent long-lasting hydrophilicity can have good hydrophilicity even after being kept at high temperatures or heat processing in the production of absorbent goods, they are preferably used to a surface sheet and/or a second sheet for absorbent articles or a sheet for lapping an absorber (core lap), such as sanitary napkins, panty liners, incontinence pads, disposable diapers and the like. Moreover, the mixed fiber spun bonded nonwoven fabric having the long-lasting hydrophilicity (5) as described later in the examples of not more than 10 [sec], particularly not more than 5 [sec] has remarkable excellent effect in the long-lasting hydrophilicity.

<Other additives>

In the present invention, to the mixed fiber spun bonded nonwoven fabrics, it is possible to add, as an optional component, various stabilizers such as a heat stabilizer, a weather stabilizer, etc., a slipping agent, an antifogging agent, a lubricant, a dye, a pigment, a natural oil, a synthetic oil and a wax.

Examples of the stabilizers are an anti-oxidant such as 2,6-di-t-butyl-4-methylphenol (BHT) etc.; a phenol anti-oxidant such as tetrakis[methylene-3-(3,5-di-t-butyl-4-hydroxyphenyl)propionate]methane, 6-(3,5-di-t-butyl-4-hydroxyphenyl)propionic acid alkyl ester, 2,2'-oxamidobis[ethyl-3-(3,5-di-t-butyl-4-hydroxy phenyl)]propionate, Irganox 1010 (hindered phenol anti-oxidant: Trade Name), etc.; and an aliphatic acid metal salt such as zinc stearate, calcium stearate, 1,2-calcium hydroxystearate, etc.

These stabilizers may be used singly or two or more may be combined for use.

<Mixed fiber spun bonded nonwoven fabric>

The mixed fiber spun bonded nonwoven fabric of the present invention is formed from a mixed fiber spun bonded nonwoven fabric which comprises 90 to 10 % by weight of the long fiber type thermoplastic resin (A) and 10 to 90 % by weight of the long fiber type thermoplastic elastomer (B) and the long fiber type thermoplastic resin (A) is at least hydrophilized.

The mixed fiber spun bonded nonwoven fabric of the present invention is a mixed fiber spun bonded nonwoven fabric having excellent initial hydrophilicity and long-lasting hydrophilicity obtainable by mixing the long fiber type thermoplastic resin (A) hydrophilized and the long fiber type thermoplastic elastomer (B) in an amount of 10 to 90 % by weight.

The long fiber type thermoplastic resin (A) for forming the mixed fiber spun bonded nonwoven fabric of the present invention needs to be hydrophilized, but the long fiber type thermoplastic elastomer (B) may be not hydrophilized.

The mixed fiber spun bonded nonwoven fabric comprises the long fiber type thermoplastic elastomer (B) in an amount of preferably not less than 20 % by weight, more preferably not less than 30 % by weight from the viewpoint of bulkiness, stretchability, flexibility, initial hydrophilicity and long-lasting hydrophilicity, and in an amount of preferably not more than 70 % by weight, more preferably not more than 60 % by weight from the viewpoint of processability (resistance to stickiness).

In the mixed fiber spun bonded nonwoven fabric of the present invention, the fiber diameter (average value) of the long fiber type thermoplastic resin (A) and that of the long fiber type thermoplastic elastomer (B) are usually not more than 50 $\mu$m, preferably not more than 40 $\mu$m, more preferably not more than 30 $\mu$m, respectively. The fiber diameter of the long fiber type thermoplastic resin (A) and that of the long fiber type thermoplastic elastomer (B) may be the same or different each other.

The mixed fiber spun bonded nonwoven fabrics of the present invention can be appropriately selected in accordance with the use. For example, when it is used as a surface material sheet of a sanitary napkin, the basis weight is not more than 120 g/m$^2$, preferably 15 to 80 g/m$^2$, more preferably 15 to 60 g/m$^2$, from the viewpoint of flexibility.

The mixed fiber spun bonded nonwoven fabric of the present invention is obtainable by using the thermoplastic resin (A) and the thermoplastic elastomer (B) by a known method of producing spun bonded nonwoven fabrics, for example, the method as described in JP-A-2004-244791.

Specifically, the thermoplastic resin (A) and the thermoplastic elastomer (B) are molten in extruders respectively, and then the molten polymers are individually introduced into a die each having a plurality of nozzles, and the thermoplastic resin (A) and the thermoplastic elastomer (B) are independently output from the individual nozzles simultaneously. Thereafter, the long fibers of the thermoplastic resin (A) and the long fibers of the thermoplastic elastomer (B) are introduced into a cooling room and cooled by cooling air, and then they are stretched (extended) by stretching air and deposited on a moving collecting surface. The melting temperature of each polymer is not particularly limited as far as it is not lower than the softening temperature or melting temperature and lower than the pyrolysis temperature of each polymer, and is determined by the polymer used. The die temperature depends on the polymer used. For example, when a propylene polymer or an olefin polymer composition of the propylene polymer and HDPE is used as the thermoplastic resin (A) and a thermoplastic polyurethane elastomer or an olefin copolymer elastomer is used as the thermoplastic elastomer (B), the die temperature is set to be usually 180 to 240°C, preferably 190 to 230°C, more preferably 200 to 225°C.

The temperature of the cooling air is not particularly limited as far as the polymer is solidified. It is generally 5 to 50°C, preferably 10 to 40 °C, more preferably 15 to 30°C. The rate of the stretching air is usually 100 to 10,000 m/min, preferably 500 to 10,000 m/min.

Before the stretch processing, the method of solidifying with press the mixed fiber spun bonded nonwoven fabric deposited by means of a nip roll is described. In this method, the roll is preferably heated. Furthermore, pre-bonding is carried out by a method of using needle punch, water jet or ultrasonic wave, thermal emboss processing using an embossing roll or hot air through. In any of the methods, the mixed fiber spun bonded nonwoven fabric is preferably co-founded lightly from the viewpoint of the touch and stretchability of the resulting surface sheet.

When heat fusion is carried out by thermal emboss processing, the embossed area proportion is 5 to 20%, preferably 5 to 10%, and the un-embossed unit area is not less than 0.5 mm$^2$, preferably 4 to 40 mm$^2$. The non-embossed unit area is the largest square area surrounded by embosses in the smallest unit of the non-embossed parts surrounded by the embossed parts. Examples of the embossed shape may include circle, oval, ellipse, square, lozenge, rectangle, square and continuances of the above shapes. When the mixed fiber spun bonded nonwoven fabric has the embossed parts in the above range, bonded points are formed in the embossed parts where the long fiber type the thermoplastic resin (A) and the long fiber type the thermoplastic elastomer (B) constituting the mixed fiber spun bonded nonwoven fabric are substantially bonded, and furthermore, in the embosses of the mixed fiber spun bonded nonwoven fabric layer, the long fiber type thermoplastic elastomer (B) having elasticity and the (stretched fiber) long fiber type thermoplastic resin (A) having elasticity substantially lower than that of the long fiber type thermoplastic elastomer (B) are present in a high degree of freedom. Therefore, when the mixed fiber spun bonded nonwoven fabric has such a structure, the residual strain thereof is decreased and it has good stretchability.

When the embossed area proportion is large, the range capable of being stretchable is decreased but the stress is improved. When the embossed area proportion is small, the range of being stretchable can be increased but when the embossing pitch is increased, the residual strain is likely somewhat increased.

Stretch processing the mixed fiber spun bonded nonwoven fabric according to the present invention, the stretched long fiber type thermoplastic elastomer (B) has a length near the length before the stretching by elastic recovery, but the long fiber type thermoplastic resin (A) almost keeps the length stretched. Therefore, since the long fiber type thermoplastic resin (A) is warped and thereby warped fibers are come out on the surface of the mixed fiber spun bonded nonwoven fabric, the mixed fiber spun bonded nonwoven fabric has more bulkiness and higher flexibility. Moreover, since the mixed fiber spun bonded nonwoven fabric also has excellent stretchability, it is suitable for surface sheets of absorbing goods having high fitting properties.

The mixed fiber spun bonded nonwoven fabric of the present invention may be laminated with other layers in accordance with various purposes. The other layers for laminating the mixed fiber spun bonded nonwoven fabric of the present invention are not particularly limited and various layers can be laminated in accordance with the purposes.

Specific examples of the other layers are knit fabrics, woven fabrics, nonwoven fabrics and films. When the mixed fiber spun bonded nonwoven fabric of the present invention is further laminated (adhered) with other layers, it is possible to employ various known methods such as heat fusion methods including heat emboss processing, ultrasonic fusion, etc; mechanical co-founding methods including needle punch, water jet, etc; and methods of using adhesives such as hot melt adhesion, urethane type adhesive, etc.; and extrusion laminating and the like.

Examples of the nonwoven fabrics for laminating on the mixed fiber spun bonded nonwoven fabric of the present invention are various known nonwoven fabrics such as spun bonded nonwoven fabrics, melt blown nonwoven fabrics, wet type nonwoven fabrics, dry type nonwoven fabrics, dry type pulp nonwoven fabrics, flash spinning nonwoven fabrics, split yarn nonwoven fabrics and the like. These nonwoven fabrics may be non-stretching nonwoven fabrics. The non-stretching nonwoven fabrics used herein have an elongation at rupture in the MD or CD of about 50% and do not cause returning stress after elongation.

The films for laminating on the mixed fiber spun bonded nonwoven fabric of the present invention are preferably breathable (moisture permeable) films capable of making the most of breathability and hydrophilicity which are the properties of the mixed fiber spun bonded nonwoven fabric of the present invention. The breathable films are various known breathable films, for example, moisture permeable films formed from thermoplastic elastomers such as polyurethane elastomer, polyester elastomer, polyamide elastomer, etc; and porous films obtainable by making films formed from inorganic or organic fine particle-containing thermoplastic resins to be porous by stretching. Preferable examples of the thermoplastic resins used in the porous films are high-pressure low density polyethylene, linear low density polyethylene, high density polyethylene, polypropylene, polypropylene random copolymer and polyolefins formed from their compositions.

< Absorbent article>

The absorbent articles of the present invention are sanitary napkins, panty liners, incontinence pads, disposable diapers and other products containing the mixed fiber spun bonded nonwoven fabrics. The absorbent articles usually have an intermediate layer made from an absorber provided between a back sheet and a liquid permeable surface sheet.

The mixed fiber spun bonded nonwoven fabric of the present invention has excellent initial hydrophilicity and long-lasting hydrophilicity, and also has flexibility and bulkiness. Therefore, it can be suitably used for a surface sheet and/or a second sheet for absorbing articles and further a sheet (core lap) for lapping absorbing materials.

EXAMPLE

The present invention will be described in more detail with reference to the following examples, but it is not limited by the examples. The physical property values in the examples and comparative examples were determined by the following methods.

(1) Basis weight [g/m$^2$]

Six specimens having a size of 200 mm (MD) x 50 mm (CD) were prepared from a (mixed fiber) spun bonded nonwoven fabric. They were prepared from any three parts of the fabric in the MD and the CD (total six places). Subsequently, the mass (g) of each specimen was measured using an electronic even balance (manufactured by Kensei Kogyo). The average value of the masses of the specimens was determined. The resultant average value was converted to the mass (g) per 1 m$^2$, and taken as a basis weight [g/m$^2$] of each nonwoven fabric by rounding to two decimal places.

(2) Thickness [$\mu$m]

Three specimens having a size of 100 mm (MD) x 100 mm (CD) were prepared from a (mixed fiber) spun bonded nonwoven fabric. They were prepared from any three parts of the fabric. Subsequently, the thickness [$\mu$m] of

each specimen was measured using a load type thickness indicator by the method as described in JIS L 1096. The average value of the thicknesses of the specimens was determined and taken as a thickness [μm] of each nonwoven fabric by rounding the value to two decimal places.

(3) Fiber diameter [μm]

A specimen having a size of 100 mm (MD) x 50 mm (CD) was prepared from a (mixed fiber) spun bonded nonwoven fabric.

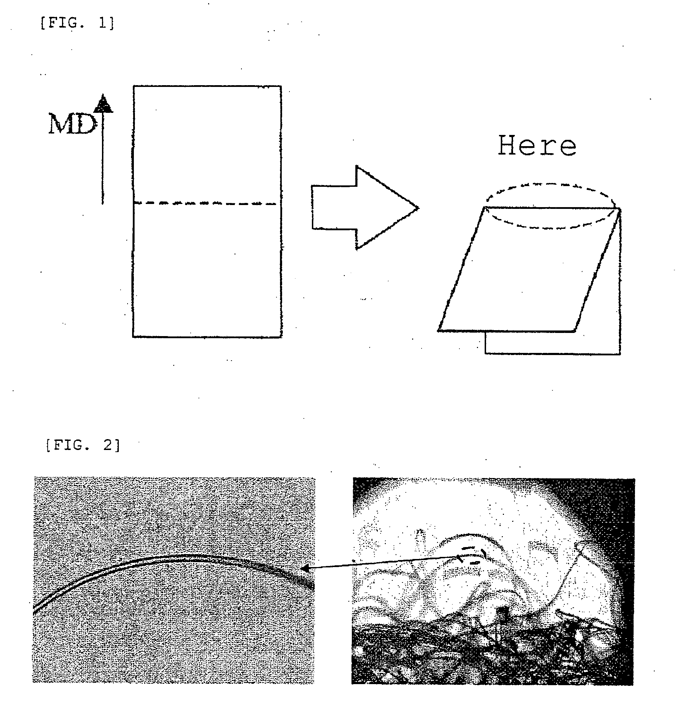

The fiber diameter of the long fiber type thermoplastic resin (A) was analyzed in the following manner. Each specimen collected was folded as shown in Fig. 1, the fiber of one side surface of the specimen folded was photographed in a magnification of 200 times as shown in Fig. 2, and the picture was analyzed by a software for measuring image size (Pixs2000 Version 2.0 manufactured by Inotex Co.). For each specimen, the diameters of 10 fibers were measured and the average value of the fiber diameter of each specimen was determined and taken as a fiber diameter [μm] of the long fiber type thermoplastic resin (A) by rounding the value to two decimal places.

The fiber diameter of the long fiber type the thermoplastic elastomer (B) was analyzed in the following manner. Each specimen collected was photographed in a magnification of 200 times without folding. Fibers having a larger fiber diameter were selected and the pictures thereof were analyzed by a software for measuring image size (Pixs2000 Version 2.0 manufactured by Inotex Co.). For each specimen, the diameters of 10 fibers were measured and the average value of the fiber diameter of the specimen was determined and taken as a fiber diameter [μm] of the long fiber type thermoplastic elastomer (B) by rounding the value to two decimal places.

(4) Initial hydrophilicity [sec]

From a mixed fiber spun bonded nonwoven fabric, three specimens having a size of 125 mm (MD) x 125 mm (CD) were prepared. The collecting places were any three ones. Subsequently, each specimen collected was measured on liquid permeation time [sec] using Lister (Lenzing Instruments Co.) in accordance with the method as described in EDANA150. 2-93.

Specifically, ten EPT EF3 filter papers (a length of 100 mm, a width of 100 mm, manufactured by Hollingsworth & Vose Company Ltd.) were used as a filter paper. Furthermore, an aqueous solution of sodium chloride (9g/liter) was used as a liquid. The solution had a surface tension of 70+/-2mN/m. Each specimen and the filters were allowed to stand at 20° C at a humidity of 65% for 24 hr. A ring stand supporting a Lister funnel which had a magnet valve part and could carry a liquid at a rate of 25 ml/(3.5+/-0.25) sec was set and a glass tube (volume of 50 ml) was prepared so that the glass tube could put in the funnel. The ten filter papers were put on a base plate of a device and each specimen was on the surface of the filter so that the top side which was contact with the skin was upper side. In this procedure, it was confirmed that an electrode in the plate was clean and also the position of the funnel was regulated so that the funnel was set at the center of the plate. The height of the funnel was regulated so that the funnel was set above the cavity plate by 5 mm (above the sample by 30 mm). It was confirmed that the electrode and the timer were connected and the timer was operated and set to be zero. Subsequently, 5 ml of artificial urine was injected to the glass tube.

Thereafter, the artificial urine was exhausted from the funnel to the cavity plate, and the time during the passing of the liquid throughout was measured in the electrode part (the measurement was carried out automatically by the timer). The average value of the liquid permeating time of each specimen was determined and taken as an initial hydrophilicity [sec] of each nonwoven fabric sample by mounting the value to two decimal places.

(5) Long-lasting hydrophilicity [sec]

From a (mixed fabric) spun bonded nonwoven fabric, three specimens having a size of 125 mm (MD) x 125 mm (CD) were collected. Three places were selected arbitrarily as a collecting place. Each collected specimen was suspended near the center of a dryer ("Tabai safety oven STS222" a length of 600 mm, a width of 600 mm, a depth of 600 mm manufactured by Espec Co.,) in such a direction that circulating air met to the specimen vertically, and the dryer was set at an inside temperature of 40° C and allowed to stand for 1 hr. Subsequently, each specimen was taken out and allowed to stand at room temperature for 1 hr. The time during the permeating of the liquid throughout [sec] was measured in the same manner as that of the initial hydrophilicity (4). The average value of the liquid permeating time of each specimen was determined and taken as long-lasting hydrophilicity [sec] of each nonwoven fabric sample by mounting the value to two decimal places.

In the following measurements on the index of repetition absorbing (6) and the liquid flow distance (7), an aqueous solution of sodium chloride (9 g/liter) having a surface tension of 70+/-2 mN was used as artificial urine. Furthermore, the measurements on the index of repetition absorbing (6) and the liquid flow distance (7) were carried out in the following two conditions of using a (mixed fiber) spun bonded nonwoven fabric prepared after the passage time of 24 hr within 48 hr (no heat treatment) from the production thereof and a (mixed fiber) spun bonded nonwoven fabric prepared by heat treating a (mixed fiber) spun bonded nonwoven fabric prepared after the passage time of 24 hr or more from the production thereof, at 80° C for 2 hr, taking out it and allowing to stand within 2 hr (heat treatment).

(6) Repetition absorption

A specimen (50 mm x 200 mm) was collected from a mixed (fiber) spun bonded nonwoven fabric. Ten filter papers (No. 2 manufactured by Advantec Co. Ltd.) were piled and the specimen was horizontally placed on the filter papers. Artificial urine was put by one drop (about 0.3 ml) from the height of about 10 mm from the specimen surface by a syringe on 10 places at a distance of 20 mm, and the number of drops which were absorbed within 2 sec was measured. This procedure was repeated three times at an interval of 3 min and the repetition absorption (time) was determined. The specimen had a larger value so that the hydrophilicity was more excellent.

(7) Liquid flow distance

From a (mixed fiber) spun bonded nonwoven fabric, a specimen (50 mm x 200 mm) was collected. Five filter papers (No. 2 manufactured by Advantec Co., Ltd.) were piled on a plate fixed and inclined at 45°. The specimen was placed on the filter papers and the ends in the length direction of the specimen were fixed on the plate together with the filters. One drop (about 0.3 ml) of artificial urine was dropped from the height of about 10 mm from the specimen surface by a syringe, and the distance between the dropping point of the drop and the place where the drop was completely absorbed was measured and taken as a liquid flow distance (mm). The specimen had a smaller value so that the hydrophilicity was more excellent.

(8) Residual strain [%]

From a (mixed fiber) spun bonded nonwoven fabric, six specimens having a size of 200 mm (MD) x 50 mm (CD) were collected. Three places were selected arbitrarily as a collecting place (total six places). Subsequently, each specimen was stretched using a universal testing machine (IM-Model 201 manufactured by Intesco Co.) in such conditions that the chuck distance was 100 mm, the tensile rate was 100 mm/min and the stretching ratio was 100%. Thereafter, the specimen was immediately returned to the original length at the same rate and the strain was measured at the returning time and taken as a residual strain [%]. The residual strain [%] of each nonwoven fabric sample was determined by determining the average value on the six points (MD, CD each 3 points) with mounting the value to one decimal place.

(9) Touch

Ten panelists confirmed the touch of a (mixed fabric) spun bonded nonwoven fabric and evaluated in accordance with the following criteria.

A: Ten panelists judged that the specimen had no stickiness and good touch among ten panelists.
B: 9 to 7 panelists judged that the specimen had no stickiness and good touch among ten panelists.
C: 6 to 3 panelists judged that the specimen had no stickiness and good touch among ten panelists.
D: 2 to 0 panelists judged that the specimen had no stickiness and good touch among ten panelists.

The analysis and evaluation on the thermoplastic polyurethane elastomer (TPU) used in the examples and the comparative examples were carried out in the following methods.

(10) Solidification starting temperature [°C]

The measurement was carried out using a differential scanning calorimeter (DSC220C) connected with SSC5200 H disk station manufactured by Seiko Instruments Inc. As a sample, about 8 mg of TPU pulverized was collected on an aluminum pan and crimped by a cover. As a reference, alumina was collected in the same manner. The sample and the reference were set in the prescribed positions in a cell and measured in a stream of nitrogen at a flow rate of 40 Nml/min. The temperature was increased from room temperature to 230° C at an increasing rate of 10° C/min and kept at the same temperature for 5 min and then decreased to -75° C at a decreasing rate of 10° C/min. At this time, the starting temperature of an exothermic peak derived from solidification of TPU was measured and taken as a solidification starting temperature (unit: °C).

(11) Number of particles insoluble in polar solvent

The measurement was carried out using Multisizer II manufactured by Beckman Coulter K.K., as a particle size distribution measuring apparatus based on an electrical sensing zone method. In a 5 1 separable flask, 3500 g of dimethylacetoamide (special grade manufactured by Wako Pure Chemical Industries) and 145.83 g of thiocyanic acid ammonium (special grade manufactured by Junsei Chemical Co., Ltd.) were weighed and dissolved at room temperature over 24 hr.

Subsequently, filtration was carried out under reduced pressure using a 1 $\mu$m membrane filter and thereby a reagent A was prepared. In a 200 cc glass bottle, 180 g of the reagent A and 2.37 g of a TPU pellet was weighed and the soluble part in TPU was dissolved over 3 hr to prepare a measuring specimen. To Multisizer II, a 100 $\mu$m aperture tube was fixed and the solvent in the device was replaced with the reagent A, and then the vacuum pressure was regulated to about 3000 mmAq. In a beaker for injecting the specimen which beaker thoroughly had been cleaned, 120 g of the reagent A was weighed and it was confirmed that the amount of the pulse generated by the blank measurement was not more than 50 pulses/min. The optimum current value and gain were set in accordance with the manual and then calibration was carried out using uncrosslinked polystyrene standard particles having a size of 10 $\mu$m. The measurement was carried out for 210 sec after 120 g of the reagent A and about 10 g of the measurement specimen were weighed in a specimen-introducing beaker thoroughly washed. The particle number counted in the measurement was divided by the TPU weight sucked in the aperture tube to determine the number of the particles insoluble in the polar solvent in TPU (unit: particles/g). The weight of TPU was determined by the following formula.

$$\text{TPU weight} = \{(A/100) \times B/(B+C)\} \times D$$

In the formula, A is a concentration (% of weight) of TPU of the specimen for measurement, B is a weight (g) of the specimen for measurement weighed in the beaker, C is a weight (g) of the reagent A weighed in the beaker and D is a weight (g) of a solution sucked in the aperture tube during the measurement (210 sec).

(12) Ratio of heat of fusion in hard domain

The heat of fusion was measured by a differential scanning calorimeter (DSC220C) connected to SSC5200H disk station manufactured by Seiko Electronic Industries. As a sample, about 8 mg of TPU pulverized was collected in an aluminum pan and crimped by a cover. As a reference, alumina was collected in the same manner. The sample and the reference were set in the prescribed positions in a cell and measured in a stream of nitrogen at a flow rate of 40 Nml/min. The temperature was increased from room temperature to 230°C at an increasing rate of 10°C/min. The total sum (a) of heat of fusion determined from an endothermic peak at a peak temperature of not lower than 90°C and not higher than 140°C and the total sum (b) of heat of fusion determined from an endothermic peak at a peak temperature of higher than 140°C and not higher than 220°C were determined and the ratio of heat of fusion of the hard domain (unit: %) was determined by the following formula.

$$\text{Ratio of heat of fusion in hard domain (\%)} = a/(a+b) \times 100$$

(13) Melt viscosity at 200°C (hereinafter referred to "melt viscosity" simply)

Using Capiro Graph (model 1C manufactured by Toyo Seiki Co. , Ltd.), the melt viscosity (unit: Pa·s) at 200°C at a shear rate of 100 sec$^{-1}$ was measured. A nozzle having a length of 30 mm and a diameter of 1 mm was used.

(14) Water content of TPU

The water content of TPU (unit: ppm) was measured in combination use of a water content measuring device (AVQ-5S manufactured by Hiranuma Sangyo Co., Ltd.) and a water content vaporizer (EV-6 manufactured by Hiranuma Sangyo Co., Ltd.). About 2 g of TPU pellets was weighed in a heating specimen pan and introduced into a heating furnace at 250°C. Vaporized moisture was led to a titration cell of the water content measuring device which residual moisture had been removed previously and titration was carried out by a Karl Fisher reagent. It was confirmed that the potential change of a titration electrode accompanied with the water content change in the cell was not caused for 20 sec to finish the titration.

(15) Shore A hardness

The hardness of TPU was measured at 23°C at a relative humidity of 50% in accordance with the method as described in JIS K-7311. The type A was used as a durometer.

<Preparation Example 1 of Thermoplastic polyurethane elastomer>

Diphenylmethane diisocyanate (hereinafter referred to MDI) was introduced into a tank in a nitrogen atmosphere and regulated with stirring in such a way that bubbles were not mixed to be at 45°C.
To a tank B, 628.6 parts by weight of polyester polyol having a number average molecular weight of 2000 (Trade Name Takelack U2024 manufactured by Mitsui Takeda Chemicals inc.) and 2. 21 parts by weight of Irganox 1010 and 77.5 parts by weight of 1,4-butane diol were introduced in a nitrogen atmosphere and regulated with stirring to be at 95°C. This mixture was taken as a polyol solution 1.
The hard segment amount calculated from these reaction raw materials was 37.1 % by weight.
Next, in a liquid transporting line with a gear pump and a flowmeter, MDI was passed through at a rate of 17.6 Kg/h and the polyol solution 1 was passed through at a rate of 42.4 Kg/h to a high speed stirrer (SM40) regulated at 120°C quantitatively and mixed with stirring at 2000 rpm for 2 min. Thereafter the mixture was passed through to a static mixer. The static mixer part was composed of connecting the first to third static mixers (temperature 230°C) that three static mixers having a tube length of 0. 5 m and an inner diameter of 20 mm were connected, the fourth to sixth static mixers (temperature 220°C) that three static mixers having a tube length of 0.5 m and an inner diameter of 20 mmØ were connected, the seventh to twelfth static mixers (temperature 210°C) that six static mixers having a tube length of 1.0 m and an inner diameter of 34 mm were connected, and the thirteenth to fifteenth static mixers (temperature 200°C) that three static mixers having a tube length of 0.5 m and an inner diameter of 38 mm were connected, in series.
The reaction product drained from the fifteenth static mixer was fed with pressure to a mono-axial extruder (diameter: 65 mm, temperature: 180 to 210°C) with a polymer filter (Trade Name: Dina filter manufactured by Nagase Co., Ltd.) on the tip through the gear pump and extruded from a strand die. After cooling with water, the extruded product was continuously pelletized. Subsequently, the resulting pellets were introduced into a dryer and dried at 100°C for 8 hr to prepare a thermoplastic polyurethane elastomer having a water content of 40 ppm. This thermoplastic polyurethane elastomer was continuously extruded by a mono-axial extruder (diameter: 50 mm, temperature:180 to 210°C) to be pelletized. The pellets were dried again at 100°C for 7 hr to prepare a thermoplastic polyurethane elastomer (TPU-1) having a water content of 57 ppm.
The starting temperature of solidifying TPU-1 was 103.7°C, the number of the particles insoluble in the polar solvent was 1, 500, 000 particles/g, the specimen prepared by injection molding has a hardness of 86A and a melt viscosity at 200°C of 1900 Pa s and ratio of heat of fusion of the hard domain was 35.2%.

Example 1

<Preparation of thermoplastic resin composition for spun bonded nonwoven fabric>

96 parts by weight of a propylene homopolymer having a MFR, as determined in accordance with ASTM D 1238, at 230°C under a load of 2.16 Kg, of 60g/10 min, a density of 0.91 g/cm$^3$, a melting point of 160°C (hereinafter referred to "PP-1") and 4 parts by weight of a high density polyethylene having a MFR, as determined in accordance with ASTM D 1238, at 1.90°C under a load of 2.16 Kg, of 5 g/10 min, a density of 0.97 g/cm$^3$, a melting point of 134°C (hereinafter referred to "HDPE"). Thereafter, with 100 parts by weight of the PP-1/HDPE mixture, 5 parts by weight (3 parts by weight in terms of a component of a hydrophilization treating agent) of a hydrophilization treating master batch (Trade Name: IRGASURF HL560 manufactured by Ciba Ltd.) (hereinafter referred to hydrophilizing agent A") containing 60 % by weight of polyoxyethylene alkylether $CH_3(CH_2)_{17}$-O-$(CH_2CH_2)_{2.5}$-H, as an ether type non-ionic surface-active agent (the Component of the hydrophilization treating agent) was mixed to prepare a thermoplastic resin composition (A-1).

<Production of Mixed fiber spun bonded nonwoven fabric>

The thermoplastic polyurethane elastomer (TPU-1) and the thermoplastic resin composition (A-1) were independently melted using a 75 mm extruder and a 50 mm extruder. Thereafter, using a spun bonding nonwoven molding machine having a spinning die (length vertical to the machine direction in the collecting surface: 800 mm), they were melt spun at a resin temperature of 210°C at a die temperature of 210°C at a cooling air temperature of 20°C at a stretching air rate of 3750 m/min by a spun bonding method, a web made from mixed long fibers containing a long fiber B of TPU-1 and a long fiber A of A-1 were deposited on the collecting surface to prepare a web of mixed fibers of fiber B and fiber A in the proportion of 40/60 (% by weight). The spinning die had a nozzle pattern that an output hole for TPU-1 and an output hole for A-1 were alternately disposed, the nozzle diameter for TPU-1 (fiber B) was 0.75 mm and the nozzle diameter for A-1 (fiber A) was 0.6 mm, the nozzle pitch was 8 mm in the longitudinal direction and 11 mm in the lateral direction and the nozzle number ratio of the nozzle for the fiber B to the nozzle for the fiber A was 1 / 1.45. The output "amount of the single hole for the fiber B was 0.60 g/(min·hole) and the output amount of the single hole for the fiber A was 0.61 g/min·hole).

The web made from the mixed long fiber deposited was nipped with a nip roll coated with a non-adhesive material disposed on a belt (linear pressure: 10 Kg/cm) and then pealed from a moving belt, and heat bonded to an emboss pattern using a heat emboss in conditions such that the area coefficient was 18%, the embossed area was 0.41 $mm^2$, the heating temperature was 105°C, the linear pressure was 70 Kg/cm, to prepare a mixed fiber spun bonded nonwoven fabric. The resulting mixed fiber spun bonded nonwoven fabric has a basis weight of 30 $g/m^2$. The resulting mixed fiber spun bonded nonwoven fabric was evaluated by the above methods. The evaluation results are shown in Table 1. The touch (9) was evaluated as "B".

Example 2

A specimen having a size of 250 mm (MD) x 200 mm (CD) was prepare from the mixed fiber spun bonded nonwoven fabric prepared in Example 1 and stretched in the MD direction in conditions such that the chuck distance was 200 mm, the tensile rate was 200 mm/min and the stretching time was 100% using a universal testing machine (model IM-201 Intesco Ltd.). After stretching, the mixed fiber spun bonded nonwoven fabric had a basis weight of 33 $g/m^2$. The resulting mixed fiber spun bonded nonwoven fabric was evaluated by the above methods. The evaluation results are shown in Table 1. The touch (9) was evaluated as "A".

Example 3

The procedure of Example 1 was repeated except that the basis weight of the mixed fiber spun bonded nonwoven fabric was 60 $g/m^2$ to prepare a mixed fiber spun bonded nonwoven fabric. The resulting mixed fiber spun bonded nonwoven fabric was evaluated by the above methods. The evaluation results are shown in Table 1. The touch (9) was evaluated as "B".

Example 4

<Preparation of thermoplastic resin composition for spun bonded nonwoven fabric>

As the ether type non-ionic surface-active agent, to 60 % by weight of an ethylene oxide adduct of eicosanol [$CH_3(CH_2)_{19}$-O-$(CH_2CH_2O)_{2.5}$-H] and 40 % by weight of a propylene homopolymer having MFR of 30 g/10 min, 0.05 part by weight of an antioxidant (Trade Name: Irgafos 168 manufactured by Ciba Co., Ltd.), and melt kneaded at 230°C and extruded to prepare a pelletized master batch (Hydrophilizing agent B).

Subsequently, 96 parts by weight of PP-1 used in Example 1 and 4 parts by weight of HDPE were mixed. Thereafter, 5 parts by weight (3 parts by weight in terms of a component of a hydrophilization treating agent) of the hydrophilizing agent B was mixed with 100 parts by weight of the PP-1/HDPE mixture to prepare a thermoplastic resin composition (A-2).

<Production of mixed fiber spun bonded nonwoven fabric>

The procedure of Example 3 was repeated except that the thermoplastic resin composition (A-2) was used in place of the thermoplastic resin composition (A-1) used in Example 3 to prepare a mixed fiber spun bonded nonwoven fabric. The resulting mixed fiber spun bonded nonwoven fabric had a basis weight of 60 $g/m^2$. The resulting mixed fiber spun bonded nonwoven fabric was evaluated by the above methods. The evaluation results are shown in Table 1. The touch (9) was evaluated as "B".

Example 5

<Preparation of thermoplastic resin composition for spun bonded nonwoven fabric>

96 parts by weight of a propylene/ethylene random copolymer having MFR, as determined in accordance with ASTM D1238, at a temperature of 230°C under a load of 2.16 Kg, of 60 g/10 min, a density of 0.91 g/cm$^3$ and a melting point of 142°C (hereinafter referred to "pp-2") and 4 parts by weight of HDPE were mixed. Thereafter, 5 parts by weight (3 parts by weight in terms of a component of a hydrophilization treating agent) of the hydrophilizing agent A was mixed with 100 parts by weight of the PP-2/HDPE, mixture to prepare a thermoplastic resin composition (A-3) .

<Production of mixed fiber spun bonded nonwoven fabric>

The procedure of Example 1 was repeated except that the thermoplastic resin composition (A-3) was used in place of the thermoplastic resin composition (A-1) used in Example 1 to prepare a mixed fiber spun bonded nonwoven fabric. The resulting mixed fiber spun bonded nonwoven fabric had a basis weight of 30 g/m$^2$. The resulting mixed fiber spun bonded nonwoven fabric was evaluated by the above methods. The evaluation results are shown in Table 1. The touch (9) was evaluated as "B".

Example 6

<Preparation of thermoplastic resin composition for spun bonded nonwoven fabric>

96 parts by weight of PP-2 used in Example 5 and 4 parts by weight of HDPE were mixed. Thereafter, 5 parts by weight (3 parts by weight in terms of a component of a hydrophilization treating agent) of the hydrophilizing agent B used in Example 4 was mixed with 100 parts by weight of the PP-2/HDPE mixture to prepare a thermoplastic resin composition (A-4).

<Production of mixed fiber spun bonded nonwoven fabric>

The procedure of Example 1 was repeated except that the thermoplastic resin composition (A-4) was used in place of the thermoplastic resin composition (A-1) used in Example 1 to prepare a mixed fiber spun bonded nonwoven fabric. The resulting mixed fiber spun bonded nonwoven fabric had a basis weight of 30 g/m$^2$. The resulting mixed fiber spun bonded nonwoven fabric was evaluated by the above methods. The evaluation results are shown in Table 1. The touch (9) was evaluated as "B".

Table 1-1

| | | Example 1 | | Example 2 | | Example 3 | |
|---|---|---|---|---|---|---|---|
| Fiber shape | | Mixed fiber | | Mixed fiber | | Mixed fiber | |
| | | Fiber B | Fiber A | Fiber B | Fiber A | Fiber B | Fiber A |
| Weight proportion (%) | | 40 | 60 | 40 | 60 | 40 | 60 |
| Polymer (wt%) | | TPU-1 (100) | PP-1 (96) | TPU-1 (100) | PP-1 (96) | TPU-1 (100) | PP-1 (96) |
| | | - | HDPE (4) | - | HDPE (4) | - | HDPE (4) |
| Hydrophilizing agent (wt%) | | - | Hydrophilizing agent A (5) | - | Hydrophilizing agent A (5) | - | Hydrophilizing agent A (5) |
| Starting temperature for solidification of TPU | | 103.7°C | | 103.7°C | | 103.7°C | |
| Particle number of components insoluble in a polar solvent in TPU | | 150x10$^4$ particles/g | | 150x10$^4$ particles/g | | 150x10$^4$ particles/g | |
| TPU shore A hardness | | 86 | | 85 | | 86 | |
| Molding method | | Spun bond | | Spun bond | | Spun bond | |
| Fusion method | | Heat embossing | | Heat embossing | | Heat embossing | |
| Stretching treatment | | No | | MD direction x 100% | | No | |
| Basis weight (gsm) | | 30 | | 33 | | 60 | |

(continued)

| | | Example 1 | | Example 2 | | Example 3 | |
|---|---|---|---|---|---|---|---|
| Fiber shape | | Mixed fiber | | Mixed fiber | | Mixed fiber | |
| | | Fiber B | Fiber A | Fiber B | Fiber A | Fiber B | Fiber A |
| Thickness (μm) | | 266 | | 331 | | 368 | |
| Fiber diameter (μm) | | 26.6 | 23.0 | 25.9 | 13.1 | 26.2 | 22.4 |
| Initial hydrophilicity (sec) | | 1.1 | | 1.5 | | 1.7 | |
| long-lasting hydrophilicity (sec) | | 1.7 | | 3.4 | | 1.2 | |
| No heat treatment | Absorption repeat (time/ 30 times) | - | | - | | 30 | |
| | Liquid flow distance (mm) | - | | - | | 9 | |
| Heat treatment | Absorption repeat (time/ 30 times) | - | | - | | 30 | |
| | Liquid flow distance (mm) | - | | - | | 9 | |
| Residual strain (%) | | 25 | | 12 | | 28 | |

Table 1-2

| | | Example 4 | | Example 5 | | Example 6 | |
|---|---|---|---|---|---|---|---|
| Fiber shape | | Mixed fiber | | Mixed | | Mixed fiber | |
| | | Fiber B | Fiber A | Fiber B | Fiber A | Fiber B | Fiber A |
| Weight proportion (%) | | 40 | 60 | 40 | 60 | 40 | 60 |
| Polymer (wt%) | | TPU-1 (100) | PP-1 (96) | TPU-1 (100) | PP-2 (96) | TPU-1 (100) | PP-2 (96) |
| | | - | HDPE (4) | - | HDPE (4) | - | HDPE (4) |
| Hydrophilizing agent (wt%) | | - | Hydrophilizing agent B (5) | - | Hydrophilizing agent A (5) | - | Hydrophilizing agent B (5) |
| Starting temperature for solidification of TPU | | 103.7°C | | 103.7°C | | 103.7°C | |
| Particle number of components insoluble in a polar solvent in TPU | | $150 \times 10^4$ particles/g | | $150 \times 10^4$ particles/g | | $150 \times 10^4$ particles/g | |
| TPU shore A hardness | | 86 | | 86 | | 86 | |
| Molding method | | Spun bond | | Spun bond | | Spun bond | |
| Fusion method | | Heat embossing | | Heat embossing | | Heat embossing | |
| Stretching treatment | | No | | No | | No | |
| Basis weight (qsm) | | 60 | | 30 | | 30 | |
| Thickness (μm) | | 369 | | 252 | | 248 | |

(continued)

| | | Example 4 | | Example 5 | | Example 6 | |
|---|---|---|---|---|---|---|---|
| Fiber shape | | Mixed fiber | | Mixed | | Mixed fiber | |
| | | Fiber B | Fiber A | Fiber B | Fiber A | Fiber B | Fiber A |
| Fiber diameter ($\mu$m) | | 25.8 | 22.0 | 26.4 | 22.4 | 26.3 | 22.0 |
| Initial hydrophilicity (sec) | | 1.5 | | 2.0 | | 2.2 | |
| long-lasting hydrophilicity (sec) | | 1.1 | | 1.4 | | 1.6 | |
| No heat treatment | Absorption repeat (time/ 30 times) | 30 | | 30 | | 30 | |
| | Liquid flow distance (mm) | 10 | | 13 | | 12 | |
| Heat treatment | Absorption repeat (time/ 30 times) | 30 | | 30 | | 30 | |
| | Liquid flow distance (nm) | 11 | | 11 | | 11 | |
| Residual strain (%) | | 29 | | 20 | | 21 | |

Example 7

The procedure of Example 5 was repeated except that a thermoplastic polyolefin elastomer (Trade Name: VISTAMAXX VM2125 manufactured by Exxon Mobil Ltd., hereinafter referred to "TPO-1") was used in place of the thermoplastic polyurethane elastomer (TPU-1) used in Example 5 to prepare a mixed fiber spun bonded nonwoven fabric. The resulting mixed fiber spun bonded nonwoven fabric had a basis weight of 30 g/m$^2$. The resulting mixed fiber spun bonded nonwoven fabric was evaluated by the above methods. The evaluation results are shown in Table 2. The touch (9) was evaluated as "B".

Example 8

The procedure of Example 6 was repeated except that TPO-1 used in Example 7 was used as a thermoplastic elastomer in place of the thermoplastic polyurethane elastomer (TPU-1) used in Example 6 to prepare a mixed fiber spun bonded nonwoven fabric. The resulting mixed fiber spun bonded nonwoven fabric had a basis weight of 30 g/m$^2$. The resulting mixed fiber spun bonded nonwoven fabric was evaluated by the above methods. The evaluation results are shown in Table 2. The touch (9) was evaluated as "B".

Comparative Example 1

With 100 parts by weight of PP-1 used in Example 1, 5 parts by weight of the hydrophilizing agent A was mixed to prepare a thermoplastic resin composition (A-2). Next, a spun bonded nonwoven fabric was prepared using the thermoplastic resin composition (A-2) singly by the method as described in Example 1. The resulting spun bonded nonwoven fabric had a basis weight of 30 g/m$^2$. The resulting spun bonded nonwoven fabric was evaluated by the above methods. The evaluation results are shown in Table 2. Since the fabric was broken at a stretching time of less than 100%, the residual strain (8) thereof could not be measured.

Comparative Example 2

A spun bonded nonwoven fabric was prepared using the thermoplastic resin composition (A-1) used in Example 1 singly by the method as described in Example 1. The resulting spun bonded nonwoven fabric had a basis weight of 30 g/m². The resulting spun bonded nonwoven fabric was evaluated by the above methods. The evaluation results are shown in Table 2.

Comparative Example 3

The procedure of Example 1 was repeated except for not using the hydrophilizing agent A to prepare a mixed fiber spun bonded nonwoven fabric. The resulting spun bonded nonwoven fabric had a basis weight of 30 g/m². The resulting spun bonded nonwoven fabric was evaluated by the above methods. The evaluation results are shown in Table 2.
Table 2-1

|  |  | Example 7 | | Example 8 | |
|---|---|---|---|---|---|
| Fiber shape |  | Mixed fiber | | Mixed fiber | |
|  |  | Fiber B | Fiber A | Fiber B | Fiber A |
| Weight proportion (%) |  | 40 | 60 | 40 | 60 |
| Polymer (wt%) |  | TPO-1 (100) | PP-2 (96) | TPO-1 (100) | PP-2 (96) |
|  |  | - | HDPH (4) | - | HDPE (4) |
| Hydrophilizing agent (wt%) |  | - | Hydrophilizing agent A (5) | - | Hydrophilizing agent B (5) |
| Starting temperature for solidification of TPU |  | - | | - | |
| Particle number of components insoluble in a polar solvent in TPU |  | - | | - | |
| TPU shore A hardness |  | - | | - | |
| Molding method |  | Spun bond | | Spun bond | |
| Fusion method |  | Heat embossing | | Heat embossing | |
| Stretching treatment |  | No | | No | |
| Basis weight (gsm) |  | 30 | | 30 | |
| Thickness (μm) |  | 253 | | 269 | |
| Fiber diameter (μm) |  | 24.9 | 21.3 | 25.3 | 21.7 |
| Initial hydrophilicity (sec) |  | 1.5 | | 1.5 | |
| long-lasting hydrophilicity (sec) |  | 1.1 | | 0.9 | |
| No heat treatment | Absorption repeat (time/30 times) | 30 | | 30 | |
|  | Liquid flow distance (mm) | 17 | | 14 | |
| Heat treatment | Absorption repeat (time/30 times) | 30 | | 30 | |
|  | Liquid flow distance (mm) | 9 | | 14 | |
| Residual strain (%) |  | 28 | | 28 | |

Table 2-2

| | | Comparative Example 1 | | Comparative Example 2 | | Comparative Example 3 | |
|---|---|---|---|---|---|---|---|
| Fiber shape | | Single fiber | | Single fiber | | Mixed fiber | |
| | | - | - | - | - | Fiber B | Fiber A |
| Weight proportion (%) | | 0 | 100 | 0 | 100 | 40 | 60 |
| Polymer (wt%) | | - | PP-1 (100) | - | PP-1 (96) | TPU-1 (100) | PP-1 (96) |
| | | - | - | - | HDPE (4) | - | HDPE (4) |
| Hydrophilizing agent (wt%) | | - | Hydrophilizing agent A(5) | - | Hydrophilizing agent A (5) | - | - |
| Starting temperature for solidification of TPU | | - | | - | | 103.7°C | |
| Particle number of components insoluble in a polar solvent in TPU | | - | | - | | $150 \times 20^4$ particles/g | |
| TPU shore A hardness | | - | | - | | 86 | |
| Molding method | | Spun bond | | Spun bond | | Spun bond | |
| Fusion method | | Heat embossing | | Heat embossing | | Heat embossing | |
| Stretching treatment | | No | | No | | No | |
| Basis weight (qsm) | | 30 | | 30 | | 30 | |
| Thickness ($\mu$m) | | 280 | | 270 | | 253 | |
| Fiber diameter ($\mu$m) | | - | 18.1 | - | 22.4 | 26.6 | 23.0 |
| Initial hydrophilicity (sec) | | 2.3 | | 2.3 | | 11.7 | |
| long-lasting hydrophilicity (sec) | | 20.2 | | 20.5 | | 13.2 | |
| No heat treatment | Absorption repeat (time/ 30 times) | 30 | | 30 | | 0 | |
| | Liquid flow distance (mm) | 11 | | 12 | | No absorbed | |
| Heat treatment | Absorption repeat (time/ 30 times) | 0 | | 0 | | 0 | |
| | Liquid flow distance (mm) | No absorbed | | No absorbed | | No absorbed | |
| Residual strain (%) | | Failure for measurement | | 96 | | 25 | |

As is clear from Tables 1 and 2, the spun bonded nonwoven fabrics which essentially comprise the propylene polymer free from the long fiber type thermoplastic polyurethane elastomer or the propylene polymer containing a small amount of high density polyethylene (Comparative Examples 1 and 2) had an improved initial hydrophilicity of 2.1 sec but do not have the long-lasting hydrophilicity at all by adding the hydrophilizing agent. On the other hand, the mixed fiber spun bonded nonwoven fabrics mixed with the long fiber.type thermoplastic polyurethane elastomer (Examples 1 to 6) and the mixed fiber spun bonded nonwoven fabrics mixed with the long fiber type polyolefin elastomer (Examples 7 and 8) each can have an excellent initial hydrophilicity of 1.1 sec to 2.2 sec and also a very excellent long-lasting hydrophilicity of 1.7 sec to 3.3 sec only by hydrophilizing the thermoplastic resin long fiber nevertheless not hydrophilizing the long fiber type thermoplastic polyurethane elastomer and the long fiber type polyolefin elastomer.

The mixed fiber spun bonded nonwoven fabric obtainable by stretching treatment (Example 2) is bulky and has a more increased thickness, low residual strain and excellent touch as compared with the mixed fiber spun bonded nonwoven fabric without stretching treatment (Example 1).

POSSIBILITY FOR INDUSTRIAL USE

The mixed fiber spun bonded nonwoven fabrics of the present invention have excellent bulkiness, initial hydrophilicity, long-lasting hydrophilicity, flexibility, resistance to fluff, stretchability and touch and low stickiness. Therefore, making the best of the properties, the mixed fiber spun bonded nonwoven fabrics are suitably used for not only sanitary goods but also medical goods, industrial materials and other materials.

**Claims**

1. A mixed fiber spun-bonded nonwoven fabric comprising 90 to 10 % by weight of a long fiber type thermoplastic resin (A) and 10 to 90 % by weight of a long fiber type thermoplastic elastomer (B) wherein at least, the long fiber type thermoplastic resin (A) is hydrophilized.

2. The mixed fiber spun-bonded nonwoven fabric according to claim 1 wherein the hydrophilization is treated by adding 0.1 to 10 parts by weight of a non-ionic surfactant to the long fiber type thermoplastic resin (A) based on 100 parts by weight of the thermoplastic resin (A).

3. The mixed fiber spun-bonded nonwoven fabric according to claim 1 or 2 wherein the thermoplastic elastomer (B) is a thermoplastic polyurethane elastomer.

4. The mixed fiber spun-bonded nonwoven fabric according to claim 3 wherein the thermoplastic polyurethane elastomer has a solidification initiating temperature, as measured by a differential scanning calorimeter (DSC), of not lower than 65°C and a particle number of a component insoluble in a polar solvent, as measured by a particle size distribution measuring device equipped with an aperture of 100 $\mu$m based on an electrical sensing zone method, of not more than 3,000,000 particles/g.

5. The mixed fiber spun-bonded nonwoven fabric according to claim 3 or 4 wherein the thermoplastic polyurethane elastomer satisfies the following formula (1):

$$a \ / \ (a \ + \ b) \ \leq \ 0.8$$

in which a is the total sum of heat of fusion, as determined by an endothermic peak present in the temperature range of 90°c to 140°C measured by DSC and b is the total sum of heat of fusion as determined by an endothermic peak present in the temperature range of not lower than 140°C and not higher than 220°C measured by DSC.

6. The mixed fiber spun-bonded nonwoven fabric according to claim 1 or 2 wherein the thermoplastic elastomer (B) is a polyolefin elastomer.

7. The mixed fiber spun-bonded nonwoven fabric according to claim 1 or 2 wherein the thermoplastic resin (A) is a polyolefin.

8. The mixed fiber spun-bonded nonwoven fabric according to claim 7 wherein the polyolefin is a propylene polymer.

9. The mixed fiber spun-bonded nonwoven fabric according to claim 7 wherein the polyolefin comprises 99 to 80 % by weight of a propylene polymer and 1 to 20 % by weight of a high density polyethylene.

10. The mixed fiber spun-bonded nonwoven fabric according to claim 8 or 9 wherein the propylene polymer is a propylene/$\alpha$-olefin random copolymer.

11. The mixed fiber spun-bonded nonwoven fabric according to any one of claims 1 to 10 wherein the long fiber type thermoplastic resin (A) is folded and appears on the surface of the mixed fiber spun-bonded nonwoven fabric.

12. A surface sheet of an absorbent article which sheet comprises a mixed fiber spun-bonded nonwoven fabric as claimed in any one of claims 1 to 11.

13. A second sheet of an absorbent article which sheet comprises a mixed fiber spun-bonded nonwoven fabric as claimed in any one of claims 1 to 11.

14. A sheet for lapping an absorber of an absorbent article which sheet comprises a mixed fiber spun-bonded nonwoven fabric as claimed in any one of claims 1 to 11.

15. An absorbent article obtainable by using a surface sheet as claimed in claim 12 and/or a second sheet as claimed in claim 13.

16. An absorbent article comprising a mixed fiber spun-bonded nonwoven fabric as claimed in any one of claims 1 to 11.


**Patentansprüche**

1. Mischfaser-Spinnvliesstoff, umfassend 90 bis 10 Gew.% eines langfaserartigen thermoplastischen Harzes (A) und 10 bis 90 Gew.% eines langfaserartigen thermoplastischen Elastomers (B), wobei mindestens das langfaserartige thermoplastische Harz (A) hydrophilisiert ist.

2. Mischfaser-Spinnvliesstoff gemäß Anspruch 1, worin die Hydrophilisierungsbehandlung durch die Zugabe von 0,1 bis 10 Gew.-Teile eines nicht-ionischen Tensids zu dem langfaserartigen thermoplastischen Harz (A), bezogen auf 100 Gew.-Teile des thermoplastischen Harzes (A), stattgefunden hat.

3. Mischfaser-Spinnvliesstoff gemäß Anspruch 1 oder 2, worin das thermoplastische Elastomer (B) ein thermoplastisches Polyurethan-Elastomer ist.

4. Mischfaser-Spinnvliesstoff gemäß Anspruch 3, worin das thermoplastische Polyurethan-Elastomer eine Temperatur, bei der die Erstarrung beginnt, gemessen mit der dynamischen Differenzkalorimetrie (DDK), von nicht weniger als 65°C und eine Partikelzahl einer Komponente, die in einem polaren Lösungsmittel unlöslich ist, gemessen mit einem Gerät zur Partikelgrößen-Verteilungsbestimmung, ausgestattet mit einer Öffnung von 100 $\mu$m und basierend auf einer elektronischen Zonenabtastmethode, von nicht mehr als 3.000.000 Partikeln/g besitzt.

5. Mischfaser-Spinnvliesstoff gemäß Anspruch 3 oder 4, worin das thermoplastische Polyurethan-Elastomer die folgende Gleichung (1) erfüllt:

$$a \;/\; (a \;+\; b) \;\leq\; 0,8$$

worin a die Endsumme der Schmelzwärme darstellt, die durch den endothermen Peak, der in einem Temperaturbereich von 90°C bis 140°C vorhanden ist, gemessen mit der DDK, bestimmt wird und b die Endsumme der Schmelzwärme darstellt, die durch den endothermen Peak, der in einem Temperaturbereich von nicht weniger als 140°C und nicht höher als 220°C, gemessen mit der DDK bestimmt wird.

6. Mischfaser-Spinnvliesstoff gemäß Anspruch 1 oder 2, worin das thermoplastische Elastomer (B) ein Polyolefin-Elastomer ist.

7. Mischfaser-Spinnvliesstoff gemäß Anspruch 1 oder 2, worin das thermoplastische Harz (A) ein Polyolefin ist.

8. Mischfaser-Spinnvliesstoff gemäß Anspruch 7, worin das Polyolefin ein Propylenpolymer ist.

9. Mischfaser-Spinnvliesstoff gemäß Anspruch 7, worin das Polyolefin 99 bis 80 Gew.% eines Propylenpolymers und 1 bis 20 Gew.% eines Polyethylens hoher Dichte umfasst.

**10.** Mischfaser-Spinnvliesstoff gemäß Anspruch 8 oder 9, worin das Propylenpolymer ein statistisches Propylen/α-Olefin-Copolymer ist.

**11.** Mischfaser-Spinnvliesstoff gemäß irgendeinem der Ansprüche 1 bis 10, worin das langfaserartige thermoplastische Harz (A) gefaltet ist und auf der Oberfläche des Mischfaser-Spinnvliesstoffes auftritt.

**12.** Oberflächensheet eines absorbierenden Gegenstands, wobei das Sheet ein Mischfaser-Spinnvliesstoff wie in einem der Ansprüche 1 bis 11 beansprucht umfasst.

**13.** Zweites Sheet eines absorbierenden Gegenstandes, wobei das Sheet ein Mischfaser-Spinnvliesstoff wie in einem der Ansprüche 1 bis 11 beansprucht umfasst.

**14.** Sheet zum Überlappen eines Absorbers eines absorbierenden Gegenstandes, wobei das Sheet ein Mischfaser-Spinnvliesstoff wie in einem der Ansprüche 1 bis 11 beansprucht umfasst.

**15.** Absorbierender Gegenstand, der unter der Verwendung eines Oberflächensheets, wie es in Anspruch 12 beansprucht ist, und/oder eines zweiten Sheets, wie es in Anspruch 13 beansprucht ist, erhältlich ist.

**16.** Absorbierender Gegenstand, umfassend einen Mischfaser-Spinnvliesstoff, wie er in mindestens einem der Ansprüche 1 bis 11 beansprucht ist.

## Revendications

**1.** Étoffe non tissée filée-liée à fibres mélangées comprenant 90 à 10% en poids d'une résine thermoplastique (A) de type à fibres longues et 10 à 90% en poids d'un élastomère thermoplastique (B) de type à fibres longues, dans laquelle au moins la résine thermoplastique (A) de type à fibres longues est hydrophilisée.

**2.** Étoffe non tissée filée-liée à fibres mélangées selon la revendication 1 dans laquelle l'hydrophilisation est traitée en ajoutant 0,1 à 10 partie(s) en poids d'un agent tensioactif non ionique à la résine thermoplastique (A) de type à fibres longues sur la base de 100 parties en poids de la résine thermoplastique (A).

**3.** Étoffe non tissée filée-liée à fibres mélangées selon la revendication 1 ou 2 dans laquelle l'élastomère thermoplastique (B) est un élastomère thermoplastique de polyuréthane.

**4.** Étoffe non tissée filée-liée à fibres mélangées selon la revendication 3 dans laquelle l'élastomère thermoplastique de polyuréthane a une température d'initiation de solidification, telle que mesurée par analyse calorimétrique à compensation de puissance (DSC), de pas moins de 65°C et un nombre de particules d'un composant insoluble dans un solvant polaire, tel que mesuré par un dispositif de mesure de distribution de tailles de particules équipé avec une ouverture de 100 μm sur la base d'une méthode par zone de détection électrique, de pas plus de 3 000 000 particules/g.

**5.** Étoffe non tissée filée-liée à fibres mélangées selon la revendication 3 ou 4 dans laquelle l'élastomère thermoplastique de polyuréthane satisfait à la formule (1) suivante :

$$a \ / \ (a + b) \ \leq \ 0,8$$

dans laquelle a est la somme totale de chaleur de fusion, telle que déterminée par un pic endothermique présent dans la plage de températures de 90°C à 140°C mesuré par DSC et b est la somme totale de chaleur de fusion telle que déterminée par un pic endothermique présent dans la plage de températures de pas moins de 140°C et pas plus de 220°C mesuré par DSC.

**6.** Étoffe non tissée filée-liée à fibres mélangées selon la revendication 1 ou 2 dans laquelle l'élastomère thermoplastique (B) est un élastomère de polyoléfine.

**7.** Étoffe non tissée filée-liée à fibres mélangées selon la revendication 1 ou 2 dans laquelle la résine thermoplastique (A) est une polyoléfine.

8. Étoffe non tissée filée-liée à fibres mélangées selon la revendication 7 dans laquelle la polyoléfine est un polymère de propylène.

9. Étoffe non tissée filée-liée à fibres mélangées selon la revendication 7 dans laquelle la polyoléfine comprend 99 à 80% en poids d'un polymère de propylène et 1 à 20% en poids d'un polyéthylène haute densité.

10. Étoffe non tissée filée-liée à fibres mélangées selon la revendication 8 ou 9 dans laquelle le polymère de propylène est un copolymère aléatoire de propylène/$\alpha$-oléfine.

11. Étoffe non tissée filée-liée à fibres mélangées selon l'une quelconque des revendications 1 à 10 dans laquelle la résine thermoplastique (A) de type à fibres longues est repliée et apparaît sur la surface de l'étoffe non tissée filée-liée à fibres mélangées.

12. Feuille de surface d'un article absorbant, laquelle feuille comprend une étoffe non tissée filée-liée à fibres mélangées selon l'une quelconque des revendications 1 à 11.

13. Deuxième feuille d'un article absorbant, laquelle feuille comprend une étoffe non tissée filée-liée à fibres mélangées selon l'une quelconque des revendications 1 à 11.

14. Feuille pour recouvrir un absorbant d'un article absorbant, laquelle feuille comprend une étoffe non tissée filée-liée à fibres mélangées selon l'une quelconque des revendications 1 à 11.

15. Article absorbant pouvant être obtenu en utilisant une feuille de surface selon la revendication 12 et/ou une deuxième feuille selon la revendication 13.

16. Article absorbant comprenant une étoffe non tissée filée-liée à fibres mélangées selon l'une quelconque des revendications 1 à 11.

EP 2 292 822 B1

[FIG. 1]

[FIG. 2]

24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H61994128853 A
- JP 2003250836 A
- JP 2002020957 A
- JP 2004073759 A
- JP 2007009403 A

- WO 2007138733 A1
- DE 2050371 A1
- JP 9119050 A
- JP 2006188804 A
- JP 2004244791 A